# EUROPEAN PATENT SPECIFICATION

(11) **EP 1 412 424 B1**
(45) Date of publication and mention of the grant of the patent: **17.10.2007**
(21) Application number: 02722419.5
(22) Date of filing: 08.04.2002
(51) Int. Cl.: C08K 5/00, C08L 1/28

(54) **IMPROVEMENTS IN OR RELATING TO MODIFIED CELLULOSE FILMS**
VERBESSERUNGEN FÜR, UND IN BEZUG AUF, MODIFIZIERTE CELLULOSEFILME
PELLICULES DE CELLULOSE MODIFIEES AMELIOREES

(30) Priority: 11.04.2001 GB 0109088
(43) Date of publication of application: 28.04.2004
(73) Proprietor: Bioprogress Technology International, Inc., Atlanta, GA 30350 (US)
(72) Inventor: AYERS, Victoria, Jane, March, Cambridgeshire PE15 8DJ (GB); NOWAK, Edward, Zbygniew, Cambridge CB4 9YJ (GB)
(74) Representative: Hill, Justin John
(86) International application number: PCT/GB2002/001646
(87) International publication number: WO 2002/083779

(56) References cited:
- EP-A- 0 391 518
- WO-A-92/11002
- US-A- 4 576 646
- DATABASE WPI Section Ch, Week 198151 Derwent Publications Ltd., London, GB; Class A96, AN 1981-93757D XP002210823 & JP 56 142208 A (ONO PHARM CO LTD), 6 November 1981 (1981-11-06)
- DATABASE WPI Section Ch, Week 199512 Derwent Publications Ltd., London, GB; Class A96, AN 1995-085335 XP002210824 & JP 07 010756 A (NIPPON IYAKUHIN KOGYO KK), 13 January 1995 (1995-01-13)

## Description

### Field of the Invention

This invention relates to films of modified cellulose materials (or cellulose derivatives), particularly films of the modified cellulose material hydroxypropyl methyl cellulose (HPMC), and uses of such films.

### Background to the Invention

Hydroxypropyl methyl cellulose is a synthetic plastics material, which is a modified form of the naturally occurring polymer cellulose. Films (or sheets or membranes) of HPMC are available commercially and have various uses, including proposals for use as wall materials of delivery capsules, i.e. capsules designed to retain and protect their contents until an intended site of delivery or conditions of delivery are encountered, at which point the content of the capsules are released. HPMC is suitable for ingestion by humans, so delivery capsules with HPMC walls find potential use as ingestible capsules, e.g. for the delivery of accurately metered doses of pharmaceutical preparations and dietary supplements, as a possible replacement the gelatin-based capsules. See, for example, WO 97/35537, WO 00/27367 and W0 01/03676.

WO 92/11002 describes an improved wet powder, edible, film-forming composition for use in coating tablets, capsules and the like, which consists essentially of powdered pigment particles, a film-forming, water soluble or water-dispersible, edible polymer and up to approximately 30% by weight of water, plasticised with previously known plasticisers such as polyethylene glycol 400, glycerine, propylene glycol, glycerine triacetate, triethyl citrate, tributyl citrate or diethyl phthalate.

JP 56142208 discloses a filmy transvaginal preparation obtained from a combination of a water-soluble base and a water-insoluble base and characterised in that a prostaglandin contained therein shows a desired, prolonged release pattern, and a production method thereof.

US 4,576,646 describes a film-forming composition for enveloping solid forms such as pharmaceutical or food products or seeds, wherein they comprise, by weight: 15 to 85% of a cellulosic film-forming substance, 10 to 70% of at least one alpha-cellulose, 5 to 30% of at least one plasticiser suitable for consumption.

When producing HPMC films, HPMC is usually treated with a plasticiser in order to impart or improve properties of flexibility to the film. Materials used as plasticisers include polyethylene glycol (PEG), monopropylene glycol, glycerol and also acetins (which acetates of glycerol).

In a typical method of making a cast HPMC film, HPMC, PEG and water are mixed to produce an aqueous solution, followed by optimal deaeration of the solution if a non-aerated film is required. The solution is then fed in a controlled manner to the surface of a continuous belt, producing a cast film of desired thickness which is fed on the belt past heating means for drying the film. The dried film is then removed from the belt and wound onto reels.

The present invention concerns novel plasticiser materials for HPMC films.

### Summary of the Invention

According to the invention, there is provided uses, films and delivery capsules as defined in claims 1-14.

In another aspect the invention provides a hydroxypropyl methyl cellulose film, comprising hydroxypropyl methyl cellulose plasticised with a plasticiser comprising lactic acid, comprising 24% by weight lactic acid and 76% by weight hydroxypropyl methyl cellulose.

The term fruit acid is a term used in the cosmetics industry to refer to certain naturally occurring alpha hydroxyl acids (AHAs) (ie. acids with a hydroxyl group on the carbon atom immediately next to the carboxyl group), and this term is used in this specification to have this meaning. Fruit acids include the following:
1. Lactic acid (or 2-hydroxypropanoic acid), which is formed by fermentation of sugars and is found in milk.
2. Citric acid, which is found in the juice of citrus fruits, and in beets, cranberries and certain other acid fruits.
3. Malic acid (or hydroxysuccinic acid), which occurs in many acid fruits, e.g. grapes, apples and gooseberries.
4. Glycolic acid (or hydroxyethanoic acid), which occurs in the juice of sugar cane and beets.
5. Tartaric acid (or 2, 3-dihydroxy butanedioic acid), which occurs in wine.
6. Hydroxy citric acid, which is found in the fruit of the Garcinia Gambogia tree.

Fruit acids, either naturally derived or synthetically produced, are all suitable for and approved for food use, so HPMC film in accordance with the invention is suitable for ingestion by humans. HPMC film in accordance with the invention can thus be used for ingestible purposes, e.g. as wall material for ingestible delivery capsules.

The currently preferred plasticiser is lactic acid, with citric acid then malic acid being the next most favoured.

It is preferred that the plasticiser is in the form of an acid rather than a salt of the acid as acids generally have better plasticising properties, although salts (including partial salts) e.g. sodium and potassium salts of the fruit acids may be used, and in particular it may be convenient to use buffered casting solutions.

The fruit acids, particularly lactic acid, are also generally found to have good plasticising properties and to be capable of producing HPMC films with certain benefits and advantages as compared with HPMC films prepared using conventional plasticisers. These benefits and advantages include the following:
a) The film thermoforms very easily, at lower temperatures and using less energy.
b) The deformed film retains its shape, i.e. the film has no memory.
c) The film readily welds to itself and seals at lower temperatures using less heat and pressure.
d) The film tastes pleasant and has a mouth watering effect.
e) The film has a high gloss appearance, improving the appearance of the finished produce.

The plasticiser may comprise one or more materials, including one or more fruit acids and/or one or more salts of a fruit acid, possibly in combination with one or more other plasticisers such as those used in the prior art. e.g. polyethylene glycol, monopropylene glycol, glycerol and acetins.

The plasticiser is suitably present in an amount in the range of 5 to 40% by weight of the total weight of the film, typically 24% by weight of the total weight of the film. One preferred film thus comprises 25% lactic acid and 76% by weight HPMC. Where a mixture of plasticizers is used, benefits may nevertheless be seen using a fruit acid, particularly lactic acid, at lower levels, say 5% by weight of the total weight of the film.

The film may include optional colourings, e.g. in the form of known food dyes such as FD and C yellow number 5, optional flavourings, artificial sweeteners, textures etc., in known manner.

The film may optionally be foamed, expanded or gasified, with small pockets of gas, e.g. air, included in the film structure in known manner.

The film typically has a thickness in the range 50 to 200µm (microns), e.g. in the range 120 to 130µm (microns), with film thickness being controllable in known manner. Films of different thickness may be suited to different uses.

The film may be made in generally conventional manner, e.g. as described above, as is well known to those skilled in the art.

Film in accordance with the invention finds particular use as wall material for delivery capsules, as discussed above, particularly for ingestible capsules. Other uses include as biodegradable packaging, water-soluble sachets, carrier material for coating flavours (with flavour incorporated in the film or in coating on the film), for enrobing tablets etc.

In a further aspect the invention thus provides a delivery capsule an enclosing wall comprising hydroxypropyl methyl cellulose film in accordance with the invention.

Such delivery capsules may be made in generally conventional manner, e.g. as disclosed in WO 97/35537, WO 00/27367 and WO 01/03676.

The invention will be further described, by way of illustration, in the following example.

### Example

A hydroxypropyl methyl cellulose film in accordance with the invention was made, having the following composition by weight:
Hyrdroxypropyl methyl cellulose 76%
Lactic acid 24%

The film was made in generally conventional manner. HPMC, in the form of a powder, was mixed with lactic acid and water to produce an aqueous solution, with stirring. The composition of the HPMC casing solution was (%w:w) HPMC 10, water 86, lactic acid (80% solution) 4. The solution was deaerated by application of a vacuum. The solution was then fed to a feed hopper, including an elongate exit slot located a small distance above the upper surface of a moving conveyor belt adjacent an end thereof, with the slot extending perpendicularly with respect to the direction of movement of the belt. The feed arrangement geometry and speed of movement of the belt were such that a layer of liquid of desired thickness was applied to the belt and was moved on the belt away from the feed hopper, forming a film. The film was passed on the belt through a hearing zone in which hot air heated the film, driving off water and so drying the film. The resulting dried, cast film was removed from the, belt and wound onto reels. The water content of the dried film was about 4% by weight in the form of bound (non-free) water. The thickness of the dried film was 120µm (microns).

The film has certain benefits and advantages as compared with films prepared using conventional plasticisers. These include the following:
a) The film has a high gloss appearance on both sides, especially the air-dried side, improving the appearance of the finished product.
b) The film thermoforms very easily, at lower temperatures and using less energy.
c) The deformed film retains its shape, i.e. the film has no memory.
d) The film readily welds to itself and seals at lower temperatures (130°C as compared with 160°C) using less heat and pressure.
e) The film tastes pleasant and has a mouth watering effect.

The resulting film is suitable for human consumption, and one use is as a wall material for ingestible delivery capsules e.g. containing a dose of a pharmaceutical preparation or a dietary supplement. Such capsules may be made using known techniques, e.g. as described in WO 97/35537, WO 00/27367 and WO 01/03676.

## Claims

1. The use of a plasticiser comprising a fruit acid or a salt of a fruit acid to plasticise a hydroxypropyl methyl cellulose film.

2. The use according to claim 1, wherein the plasticiser comprises one or more of lactic acid, citric acid, malic acid, glycolic acid, tartaric acid and hydroxy citric acid.

3. The use according to claim 2 wherein the plasticiser comprises one or more of lactic acid, malic acid, and glycolic acid.

4. The use according to claim 2, wherein the plasticiser comprises lactic acid.

5. The use according to claim 1 or 4, wherein the plasticiser is present in an amount in the range 5 to 40% by weight of the total weight of the film.

6. The use according to claim 4, comprising by weight of lactic acid is 24% and the composition by weight hydroxypropyl methyl cellulose is 76%.

7. The use according to any one of the preceding claims, wherein the film is foamed, expanded or gasified.

8. The use according to any one of the preceding claims, wherein the film has a thickness in the range 50 to 200 µm (microns).

9. The use according to any preceding claim, wherein said film is suitable for ingestion by humans.

10. The use according to any preceding claim wherein said plasticiser comprising a fruit acid or a salt of a fruit acid is a fruit acid.

11. A hydroxypropyl methyl cellulose film, comprising hydroxypropyl methyl cellulose plasticised with a plasticiser comprising lactic acid, comprising 24% by weight lactic acid and 76% by weight hydroxypropyl methyl cellulose.

12. A film according to claim 11, wherein the film is foamed, expanded or gasified.

13. A film according to claim 11 or 12, wherein the film has a thickness in the range 50 to 200 µm (microns).

14. A delivery capsule having an enclosing wall comprising a film in accordance with any one of claims 11, 12 or 13.

## Patentansprüche

1. Verwendung eines Weichmachers, umfassend eine Fruchtsäure oder ein Salz einer Fruchtsäure, um einen Hydroxypropylmethylcellulosefilm weichzumachen.

2. Verwendung gemäß Anspruch 1, wobei der Weichmacher eine oder mehrere von Milchsäure, Zitronensäure, Äpfelsäure, Glycolsäure, Weinsäure und Hydroxyzitronensäure umfaßt.

3. Verwendung gemäß Anspruch 2, wobei der Weichmacher eine oder mehrere von Milchsäure, Äpfelsäure und Glycolsäure umfaßt.

4. Verwendung gemäß Anspruch 2, wobei der Weichmacher Milchsäure umfaßt.

5. Verwendung gemäß Anspruch 1 oder 4, wobei der Weichmacher in einer Menge in dem Bereich von 5 bis 40 Gew.-%, bezogen auf das Gesamtgewicht des Films, vorliegt.

6. Verwendung gemäß Anspruch 4, umfassend 24 Gew.-% Milchsäure und 76 Gew.-% Hydroxypropylmethylcellulose.

7. Verwendung gemäß einem der vorhergehenden Ansprüche, wobei der Film geschäumt, expandiert oder gasifiziert ist.

8. Verwendung gemäß einem der vorhergehenden Ansprüche, wobei der Film eine Dicke in dem Bereich von 50 bis 200 µm (Mikrometer) aufweist.

9. Verwendung gemäß einem vorhergehenden Anspruch, wobei der Film zur Verdauung durch Menschen verwendbar ist.

10. Verwendung gemäß einem vorhergehenden Anspruch, wobei der Weichmacher, der eine Fruchtsäure oder ein Salz einer Fruchtsäure umfaßt, eine Fruchtsäure ist.

11. Hydroxypropylmethylcellulosefilm, umfassend Hydroxypropylmethylcellulose, weichgemacht mit einem Milchsäure-umfassenden Weichmacher, umfassend 24 Gew.-% Milchsäure und 76 Gew.-% Hydroxypropylmethylcellulose

12. Film gemäß Anspruch 11, wobei der Film geschäumt, expandiert oder gasifiziert ist.

13. Film gemäß Anspruch 12, wobei der Film eine Dicke in dem Bereich von 50 bis 200 µm (Mikrometer) aufweist.

14. Verabreichungskapsel mit einer umschließenden Wand, umfassend einen Film gemäß einem der Ansprüche 11, 12 oder 13.

## Revendications

1. Utilisation d'un plastifiant comprenant un acide de fruit ou un sel d'un acide de fruit destiné à plastifier un film d'hydroxypropyl méthyl cellulose.

2. Utilisation selon la revendication 1, dans laquelle le plastifiant comprend un ou plusieurs acides parmi l'acide lactique, l'acide citrique, l'acide malique, l'acide glycolique, l'acide tartrique, et l'acide hydroxycitrique.

3. Utilisation selon la revendication 2, dans laquelle le plastifiant comprend un ou plusieurs acides parmi l'acide lactique, l'acide malique, et l'acide glycolique.

4. Utilisation selon la revendication 2, dans laquelle le plastifiant comprend de l'acide lactique.

5. Utilisation selon la revendication 1 ou 4, dans laquelle le plastifiant est présent en une quantité de 5 à 40 % en poids du poids total du film.

6. Utilisation selon la revendication 4, comprenant 24 % en poids d'acide lactique et la composition est de 76 % en poids d'hydroxypropyl méthyl cellulose.

7. Utilisation selon l'une quelconque des revendications précédentes, dans laquelle le film est transformé en film mousse, expansé ou gazéifié.

8. Utilisation selon l'une quelconque des revendications précédentes, dans laquelle le film présente une épaisseur de 50 à 200 µm (microns).

9. Utilisation selon l'une quelconque des revendications précédentes, dans laquelle ledit film est adapté pour être ingéré par des humains.

10. Utilisation selon l'une quelconque des revendications précédentes, dans laquelle ledit plastifiant comprenant un acide de fruit ou un sel d'un acide de fruit est un acide de fruit.

11. Film d'hydroxypropyl méthyl cellulose, comprenant de l'hydroxypropyl méthyl cellulose plastifié avec un plastifiant comprenant de l'acide lactique, comprenant 24 % en poids d'acide lactique et 76 % en poids d'hydroxypropyl méthyl cellulose.

12. Film selon la revendication 11, dans lequel le film est transformé en film mousse, expansé ou gazéifié.

13. Film selon la revendication 11 ou 12 dans lequel le film présente une épaisseur de 50 à 200 µm (microns).

14. Capsule d'apport ayant une paroi de fermeture comprenant un film selon l'une quelconque des revendications 11, 12 ou 13.
